# EUROPEAN PATENT SPECIFICATION

(11) **EP 2 305 268 B1**
(45) Date of publication and mention of the grant of the patent: **20.04.2016**
(21) Application number: 08772975.2
(22) Date of filing: 24.06.2008
(51) Int. Cl.: A61K 31/575, A61K 36/076, A61P 37/08, A61P 11/06

(54) **A PHARMACEUTICAL COMPOSITION FOR TREATING DISEASE CAUSED BY IMMUNE DISTURBANCE AND THE EXTRACT FROM TUCKAHOE**
PHARMAZEUTISCHE ZUSAMMENSETZUNG ZUR BEHANDLUNG VON ERKRANKUNGEN, DIE DURCH IMMUNSTÖRUNGEN VERURSACHT WERDEN, UND GOLDKEULENEXTRAKT
COMPOSITION PHARMACEUTIQUE DESTINÉE AU TRAITEMENT DE MALADIES PROVOQUÉES PAR UNE PERTURBATION IMMUNITAIRE ET EXTRAIT DE PELTANDRA

(43) Date of publication of application: 06.04.2011
(73) Proprietor: Sinphar Tian-li Pharmaceutical Co., Ltd. (Hangzhou), Yuhang Economic Development Zone Hangzhou Zhejiang 311100 (CN)
(72) Inventor: LIN, Hang-Ching, Taipei Taiwan (CN); WU, Wen-Mein, Taipei Taiwan (CN)
(74) Representative: Grünecker Patent- und Rechtsanwälte PartG mbB
(86) International application number: PCT/CN2008/001218
(87) International publication number: WO 2009/155730

(56) References cited:
- EP-A1- 1 498 130
- EP-A1- 1 535 619
- WO-A2-2006/007538
- CN-A- 1 548 048
- DATABASE MEDLINE [Online] US NATIONAL LIBRARY OF MEDICINE (NLM), BETHESDA, MD, US; April 2000 (2000-04), LIN X ET AL: "[Study on treatment of eczema by Chinese herbal medicine with anti-type IV allergic activity].", XP002666235, Database accession no. NLM11789261 & ZHONGGUO ZHONG XI YI JIE HE ZA ZHI ZHONGGUO ZHONGXIYI JIEHE ZAZHI = CHINESE JOURNAL OF INTEGRATED TRADITIONAL AND WESTERN MEDICINE / ZHONGGUO ZHONG XI YI JIE HE XUE HUI, ZHONGGUO ZHONG YI YAN JIU YUAN ZHU BAN APR 2000 LNKD- PUBMED:11789261, vol. 20, no. 4, April 2000 (2000-04), pages 258-260, ISSN: 1003-5370
- TODA S ET AL: "Effects of the Chinese Herbal Medicine 'Saiboku-to' on Histamine Release from and the Degranulation of Mouse Peritoneal Mast Cells Induced by Compound 48/80", JOURNAL OF ETHNOPHARMACOLOGY, ELSEVIER SCIENTIFIC PUBLISHERS LTD, IE, vol. 24, 1 January 1988 (1988-01-01), pages 303-309, XP002995192, ISSN: 0378-8741, DOI: 10.1016/0378-8741(88)90159-6
- TAI TAKAAKI ET AL: "Isolation of lanostane-type triterpene acids having an acetoxyl group from sclerotia of Poria cocos", PHYTOCHEMISTRY (OXFORD), vol. 40, no. 1, 1995, pages 225-231, XP002666236, ISSN: 0031-9422
- RÍOS JOSÉ-LUIS: "Chemical constituents and pharmacological properties of Poria cocos.", PLANTA MEDICA MAY 2011 LNKD- PUBMED:21347995, vol. 77, no. 7, May 2011 (2011-05), pages 681-691, XP002666237, ISSN: 1439-0221
- SONG ZHIQI ET AL.: 'A Study of Suppressive Effects of Cortex Phellodendri. Poria. And Fructus Gardeniae on Delayed Hypersensitivity Reaction.' CHIN J DERM VENEREOL (CHINESE) vol. 11, no. 3, May 1997, pages 143 - 144, XP008140304
- ZHONG ZHAOJIN ET AL.: 'Study Progresses in Triterpenes of Poria cocos and Its Pharmacology.' CHINESE TRADITIONAL PATENT MEDICINE (CHINESE). vol. 23, no. 1, January 2001, pages 58 - 62, XP008140264
- DATABASE WPI Derwent Publications Ltd., London, GB; AN 1985-277495, XP008139283 & DE 34 16 112 A1 (ROECAR HOLDINGS NV) 31 October 1985

## Description

### FIELD OF THE INVENTION

The present invention relates to lanostane compounds for use in treating allergies.

### DESCRIPTION OF PRIOR ART

Immunoglobulin E (IgE) is one class of immunoglobulin (or "antibody") molecule. IgE is present in human serum in lower concentrations than the other immunoglobulins: IgG, IgM, IgA, and IgD. IgE is thought to have a role in protection against parasites, but has never been definitively established as playing a necessary, or even a beneficial role, at least in developed countries, where parasite infections are not a significant problem. IgE is well known as the mediator of immediate-type hypersensitivity allergic reactions, including allergic rhinitis ("hay fever"), asthma, urticaria and food and drug allergies.

In IgE-mediated allergic reactions, IgE, after it is secreted by B cells, binds through its Fc portion to the FcεRI receptors, which are present on the surface of basophils, mast cells and Langerhans cells. If the IgE bound to the surface of these cells now contacts and binds an allergen, this causes a cross-linking of the bound IgE molecules and hence the underlying receptors, and triggers the release of pharmacologic mediators, such as histamine, serotonin, leukotrienes and the slow reacting substance of anaphylaxis. These mediators cause the pathologic manifestations of allergic reactions.

Some patients with a history of some or all of the IgE-mediated allergic conditions also suffer from a painful skin condition called atopic dermatitis.

Among the allergic disease taking asthma as an example the immune reaction time can be divided into an immediate asthmatic response and a late asthmatic response. The immediate asthmatic response is a response caused by the inflammatory mediators released from the mast cells, which occurs within 15 to 30 minutes following the contact of the allergen. The allergen identifies and binds to IgE bound to the mast cells, when the patient is exposed to the same allergen again, activating the mast cell, and granules in the cells undergoing degranulation, so that more inflammatory substances are released, including histamine, leukotrienes, cytokines such as IL-2, IL-4, IL-5 and GM-CSF, and chemoattractant factors, etc., and thus the permeability of the vessels increases and the smooth muscle of the airway contracts. These inflammatory substances, cytokines and chemoattractant factors not only affect the immediate asthmatic response, but the late inflammatory response.

The response caused by eosinophils and neutrophils pertains to the late response. 4 to 6 Hours after the immediate response the cytokines and chemoattractant factors released from the mast cells will attract the inflammatory cells such as eosinophils and neutrophils, resulting in infiltration. Further, Cytokines such as Eotaxin secreted from epithelial cells, endothelial cells and fibroblast cells are the major portion of the chemoattractant factors, which also cause eosinophils or Th2 immune cells moving to the inflammatory sites at the bronchi in the lungs. Many inflammatory proteins such as MBP (Major Basic Protein), ECP (Eosinophil Cationic Protein), Eosinophil Derived Neurotoxin, EPO (Eosinophil Peroxidase), and leukotrienes, etc. will be secreted from eosinophils, when they are activated. These inflammatory proteins irritate the smooth muscle of the airway to contract, increasing the permeability of the vessels, and causing the airway edema, so that the epithelial tissues of the airway are hurt directly, and thus the epithelial cells lose their integrity. The airway secretes too much mucus, which not only blocks or narrows the airway, but causes infiltration of neutrophils and degranulation of the mast cells, thereby increasing infiltration of eosinophils. As a result, the degree of asthma is more severe.

The applicant of the present application in Taiwan Invention Patent Application No. 92113393 (publication No. 200425900, published on December 1, 2004) discloses a pharmaceutical composition useful in enhancing immunity of human body. The composition contains potent components of lanostane compounds. A *Poria* extract for enhancing immunity of human body is also disclosed, which contains 5-60 wt% of the lanostane compounds and is devoid of secolanostane. The extract is prepared from metabolite, sclerotium, or fermentation product of *Poria cocos* (Schw) Wolf.

A substance capable of treating an allergy is an important issue not only to researchers but also to ordinary people alike. It has not been reported so far that a lanostane compound is potent in treating an allergy. EP 1 535 619 discloses the use of lanostane compounds for enhancing immunity.

### SUMMARY OF THE INVENTION

The problem of the present invention is to provide a novel use of lanostane compounds. Said problem is solved by a lanostane having the following chemical formula (I) or a pharmaceutically acceptable salt thereof for use in the treatment of allergy in a mammal, 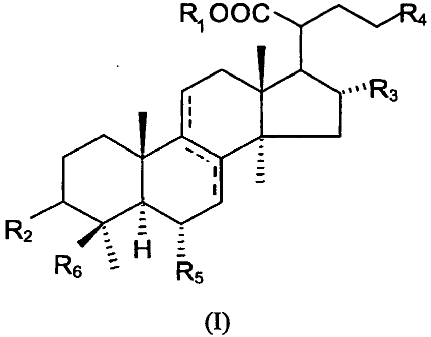 wherein R₁ is either H or CH₃; R₂ is OCOCH₃, =O or OH; R₃ is H or OH; R₄ is -C(=CH₂)-C(CH₃)₂Rₐ, in which Rₐ is H or OH, or -CH=C(CH₃)-R_{b}, in which R_{b} is CH₃ or CH₂OH; R₅ is H or OH; and R₆ is CH₃ or CH₂OH.

Preferably, the allergy is allergic rhinitis, allergic conjunctivitis, allergic asthma, atopic dermatitis, gullet allergy, atopic eczema, or rheumatoid arthritis. Most preferably, said allergy is allergic asthma.

Preferably, the lanostane having the following chemical formula (I) is 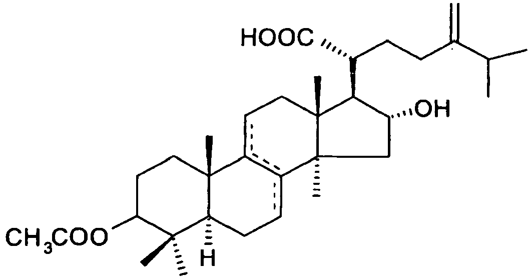 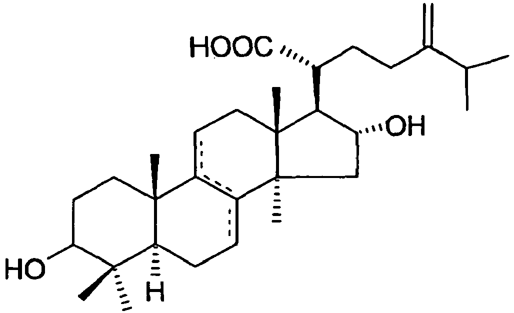 or

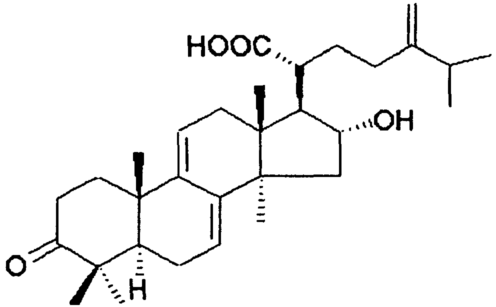

In an embodiment of the present invention a *Poria* extract is used as a source of said lanostane (I). Said *Poria* extract comprises 1-60% of the lanostane (I) by weight of the extract, and being substantially devoid of secolanostane.

Preferably, said *Poria* extract is prepared by a method comprising the following steps:
a) extracting metabolites, fermentation products or sclerotium of *Poria cocos* (Schw) Wolf by water, methanol, ethanol, or a mixed solvent thereof;
b) concentrating the resulting liquid extract from step a);
c) introducing the resulting concentrated substance from step b) into a silica gel column;
d) eluting the silica gel column with an eluent having a low polarity, and collecting the resulting eluate; and
e) concentrating the eluate to form a concentrated eluate.

Preferably, the concentrated eluate from step e) has a chromatographic value, Rf, not less than 0.1 in accordance with a thin layer chromatography, which is developed by a mixed solvent of dichloromethane : methanol = 96:4 and is detected by an ultraviolet lamp and iodine vapor.

Preferably, the extraction in step a) is carried out by using 95% ethanol.

Preferably, the extraction in step a) comprises extracting metabolites, fermentation products or sclerotium of *Poria cocos* (Schw) Wolf by boiling water; adding a base to the resulting extraction aqueous solution until a pH value thereof is 9-11; recovering the basic aqueous solution; adding an acid to the basic aqueous solution until a pH value thereof is 4-6 to form a precipitate; recovering the precipitate; extracting the precipitate with ethanol; and recovering a liquid extract.

Preferably, the concentrated substance resulted from step b) is further extracted with a two-phase solvent containing methanol and n-hexane in a volumetric ratio of 1:1, a methanol layer is separated from the two-phase solvent extraction mixture, and the methanol layer is concentrated to form a concentrate, which is used as a feed to the silica gel column in step c).

Preferably, the low polarity eluent in step d) is a mixed solvent containing dichloromethane and methanol in a volumetric ratio of 96.5:3.5.

Preferably, said *Poria* extract comprises 5-35% of the lanostane (I). Further preferred embodiments of the present invention are set forth in the dependent claims.

### BEST MODES OF EMBODYING THE INVENTION

In the following experiments conducted herein the mice were induced to become allergic by using ovalbumin (OVA) as an allergen, and the mice were confirmed suffering the induced asthma from the appearance of OVE-specific IgE antibody in the mice. Mice in different groups were fed with a *Poria* extract or pure lanostane compound during the experimental period, and each mouse was subjected to an airway hyperresponsiveness test after one-month feeding, from which an important index reflecting the severe degree of the asthma of the mouse was determined. The inventors of the present invention also observed different immune cells in bronchoalveolar lavage fluid (BALF) of the mice as to whether they particularly eosinophils and neutrophils were affected. Further, a chemoattractant factor, Eotaxin, was an important observation factor in the present invention for observing whether the secretion of cytokines changed dramatically. The observations of the above-mentioned three pharmacological or inflammatory substances, airway hyperresponsiveness, inflammatory cells and chemoattractant factor (Eotaxin), reveal that the *Poria* extract and lanostane compounds are an excellent medicine for the prophylaxis and treatment of asthma.

An extract of *Poria* for enhancing nutrient uptake by mammals (for example, humans) disclosed in the present invention can be prepared by a process similar to that disclosed in the aforesaid Taiwan Invention Patent Publication No. 200425900, which includes extracting *Poria cocos* (Schw) Wolf with the conventional extraction methods to obtain a crude extract, separating the crude extract by chromatography into a low polarity fraction of lanostane (with an eluent of dichloromethane : methanol of 96 : 4) and a high polarity fraction of secolanostane (with eluents of dichloromethane : methanol of 90 : 10, and 0 : 100), wherein the lanostane fraction is detected by a thin layer chromatography having a chromatographic value, Rf, not less than 0.1 in accordance, when it is developed by a mixed solvent of dichloromethane : methanol = 96:4; the Rf is less than 0.1 for the secolanostane fraction. Several lanostanes are separated from the lanostane fraction by subjecting the lanostane fraction to silica gel column chromatography eluted, wherein the eluents used are dichloromethane : methanol = 97 : 3 to 95 : 5.

The following examples are provided for describing the present invention in further details, but should not be used to limit the scope of the present invention.

Percentages and other amounts referred to in this specification are by weight unless indicated otherwise. Percentages are selected from any ranges used to total 100%.

### Example 1:

A *Poria* powder was made of 30 kilograms of the China-grown *Poria cocos* (Schw) Wolf. The *Poria* powder was extracted with 120 L 95% alcohol for 24 hours. The mixture was filtered to obtain a filtrate. The residue was extracted and filtered for another three cycles. The filtrates were combined and concentrated to bring about a dried extract in amount of 265.2 grams. The dry extract was undergone a distribution extraction with a two-phase extraction agent (n-hexane : 95% methanol = 1:1), and the methanol layer was removed therefrom, which is then concentrated to obtain a dry solid in an amount of 246.9 grams. A separation of the dry solid was carried out by means of a silica gel column, which was filled with silica gel 10-40 times of the weight of the dry solid. The silica gel having a diameter of 70-230 mesh was made by Merck Corporation with a code of Silica Gel 60. The column was eluted by the following eluates in sequence: a mixed solvent of dichloromethane : methanol = 96:4; a mixed solvent of dichloromethane : methanol = 90:10, and pure methanol. The eluates were tested by the thin layer chromatography (TLC), wherein an ultraviolet lamp and iodine vapor were used for detecting, and a mixed solvent of dichloromethane : methane = 96:4 was used as a developing liquid. The eluates having similar constituents in the TLC were combined.

The elution carried out with the mixed solvent of dichloromethane : methanol = 96:4 resulted in a PCM portion in amount of 78 grams. The PCM shows 6 trace points in the thin layer chromatography. The resulting eluates from the elutions carried out with the eluents of dichloromethane : methanol = 90:10 and pure methanol were combined to obtain a PCW portion in amount of 168 grams.

The PCM portion was further separated by means of an eluent of dichloromethane : methanol = 96.5:3.5 and the same silica gel column to obtain purified lanostane components of K1 (K1-1 and K1-2), K2 (K2-1 and K2-2), K3, K4, K4a, K4b, K5, K6a and K6b. Further details of the separation steps and identification analysis data can be seen in the aforesaid Taiwan Invention Patent Publication No. 200425900.

The aforesaid K1 to K6b compounds have the following structures:

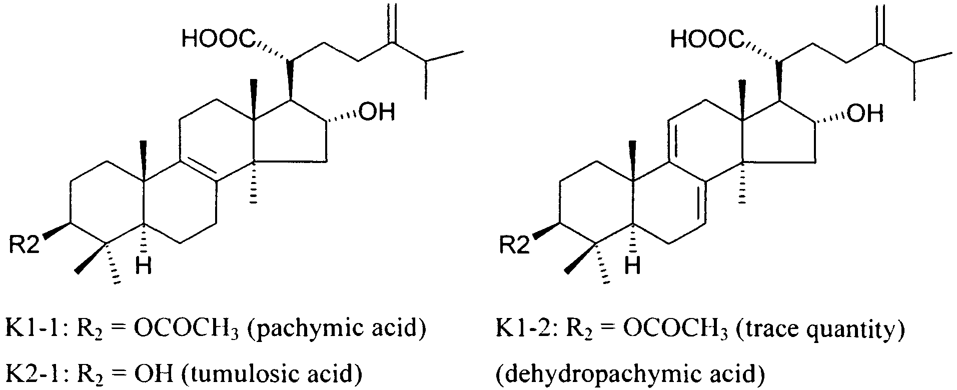

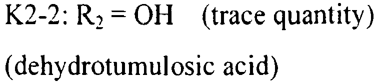

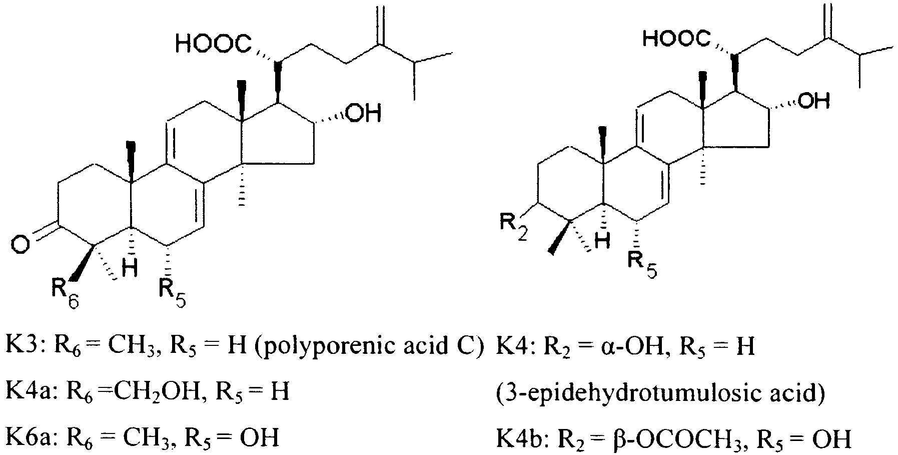

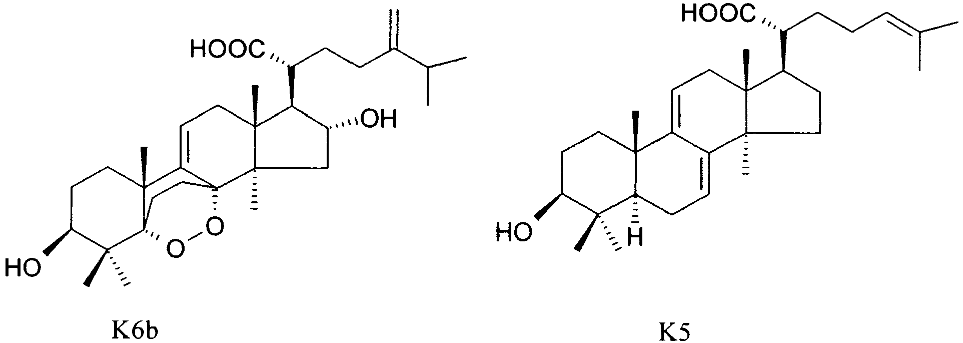

The amounts of the lanostane compounds K1 to K6b separated from the PCM portion are listed in the table below. The PCM portion contains approximately 15 wt% of the lanostane compounds K1 to K6b.

| K1 | K2 | K3 | K4 | K4a | K4b | K5 | K6a | K6b |
|---|---|---|---|---|---|---|---|---|
| 3.0 g | 6.2 g | 1.93 g | 0.55 g | 66 mg | 86.8 mg | 47.6 mg | 21.4 mg | 90.7 mg |

### Example 2:

100 kg of *Poria* was boiled with 800 kg of water for 3 hours, then left for cooling to 50° C and a pH value thereof was adjusted to pH 11 by using a 5N NaOH solution, followed by stirring the resulting solution for 3 hours. A centrifugation machine was used to separate the liquid from the solid, followed by adding another 800 kg of water to the separated solid. The aforesaid procedures were repeated, including adjusting pH value with NaOH to pH 11, stirring, and removing the solid by centrifugation. The two resulting liquids were combined, and then vacuum concentrated to a solution of 100 kg at 50° C, followed by the adjustment of pH value to pH 6.5 by using 3N HCl so as to produce a precipitate. Said precipitate was separated from the solution, subsequently rinsed with 40 L H₂O, and centrifuged in order to recover the precipitate; the precipitate was sprayed dry with 8 L of water, which yielded 380 g of powder. Afterwards, the powder was extracted three times by using 4 L of alcohol, and the extraction solutions were combined and concentrated to result in 238.9 g of alcohol extract. The 238.9 g of alcohol extract was proved containing no secolanostane compound by the TLC analysis in Example 1, and then was subject to HPLC separation, which gave 185.93 mg of K2, 20.34 mg of K3, 15.82 mg of K4, and 4.52 mg of K1 per gram of the extract. In other words, each gram of the extract has approximately 226.07 mg of lanostane compounds.

### Example 3:

### Experiments of treating the asthmatic mice with Poria extract or pure lanostane compounds

In this example the mice were induced to become allergic by using ovalbumin (OVA) as an allergen, and the mice were confirmed suffering the induced asthma from the appearance of OVE-specific IgE antibody in the mice. Mice in different groups were fed with a *Poria* extract or pure lanostane compound during the experimental period, and each mouse was subjected to an airway hyperresponsiveness test after one-month feeding, from which an important index reflecting the severe degree of the asthma of the mouse was determined. In this example different immune cells in bronchoalveolar lavage fluid (BALF) of the mice were also observed as to whether they particularly eosinophils and neutrophils were affected. Further, a chemoattractant factor, Eotaxin, was an important observation factor in this example for observing whether the secretion of cytokines changed dramatically.
Experimental method:
(1) Experimental animal: Inbred BALB/c female mice were maintained by standard laboratory chow *ad libitum.* Room temperature was maintained at 19-24°C and relative humidity at 50-70%. Animal experiments were performed according to the Guidelines for the Care and Use of Laboratory Animals, Fu-Jen University, Taiwan. BALB/c mice were divided into 10 groups each of which had 8 mice.
(2) Establish of the experimental asthma model of animals: The immunization protocol was similar and modified according to that described previously (Sy, L. B, et al. Propolis extracts exhibit an immunoregulatory activity is an ova-sensitized airway inflammatory animal model. Int Immunopharm 6:1053-1060. (2006). Briefly, the BALB/c mice were immunized with an intraperitoneal injection of 10 µg/ml and 30 µg/ml OVA (Albumin, chicken egg, A-5503, Sigma) with the 2 mg adjuvant aluminium hydroxide (Al(OH)₃, 77161, Pierce) at 8 and 10 weeks of age, respectively. All mice were exposed to inhalate exposure to 8 ml of 2% OVA aerosols for a period of 20 minutes by placing them in a chamber using an ultrasonic nebulizer (DeVilbiss Pulmo-Aide, 5650D, USA), after receiving the second intraperitoneal injection.
(3) Grouping of the experimental animal: The 10 groups of OVA-sensitized asthmatic mice were separated into a non-treatment asthma group (As); treatment groups with the Poria extract prepared in Example 2 and pure compounds K1, K2 and K3 (1PCE, 1K1, 1 K2, 1K3, 2PCE, 2K 1, 2K2, and 2K3); and the control drug group (Pred). Each mouse in the treatment group of 1PCE group received daily 0.0372 mg of the ethanol extract PCE prepared in Example 2 (1PCE). 2PCE means mice were fed with two times of the dosage of PCE (0.0744 mg) of the 1PCE group. Each mouse in the treatment groups of 1K1, 1K2 and 1 K3 received daily 0.0087 mg of K1, K2 and K3, respectively. 2K1, 2K2, and 2K3 mean mice werer fed with two times of the dosage of K1, K2 and K3 (0.0174 mg) in comparison with the 1K1, 1 K2 and 1K3 groups. Each mouse of the Pred group had been administrated with 0.1 mg of Prednisolone per day for 5 continuous days before the mouse was sacrificed. Above PCE, K1, K2, K3 and Prednisolone compounds were dissolved by 95% ethanol firstly, and finally in Phosphate Buffer Solution (PBS). Each mouse was administered 0.4 mL of total volume each time by gavage.
(4) Airway hyperresponsiveness test:
   An airway hyperresponsiveness (AHR) value increases proportionally with the severe degree of asthma, and thus a decrease of AHR value is an important index for determining whether asthma is being released. Each mouse in all groups was subjected to the AHR test next day following the 29^{th} day of administering in the treatment groups on which the last inhalate exposure was conducted. The AHR was tested with Buxco system (Biosystem XA; Buxco Electronics Inc. Sharon, CT, USA), wherein the mouse was placed in a chamber, and aerosol of PBS or methacholine of different concentrations (25 mg/ml, and 50 mg/ml) was introduced into the chamber by using an ultrasonic nebulizer. Three minutes after the introduction an average value of AHR per minute was recorded. The contraction of airway induced by methacholine was more significant when the concentration of methacholine was increased. The value of Penh (pause of enhance) was calculated by collecting data derived from transducer (differential pressure transducer; Buxco) and preamplifier (MAX □, Buxco) in the system. The relative increase ratio of Penh was calculated as Penh% = Penh_{methacholine}/Penh_{PBS}, wherein Penh_{methacholine} is the average value of Penh after the mice inhale 3 minutes
   of methacholine aerosol, and Penh_{PBS} is the average value of Penh after the mice inhale 3 minutes of PBS aerosol.
(5) Bronchoalveolar lavage fluid (BALF) and lung histology:
   As all groups of mice had finished of AHR analysis, they were sacrificed on next day. The lung was immediately lavaged via the trachea cannula (18 GA, angiocath, B.D.) with 1 ml of Hank's balanced salt solution (HBSS, SH30016.01, Hyclone, USA), which contained 2% fetal bovine serum (FBS) and 2 mM 2Na-EDTA for three times. About 3 ml of BALF were collected. The first collected of BALF was centrifuged at 1500 rpm for 5 min at 4°C. Collected supernatants of BALF were stored at -20°C for determining the levels of cytokines. The second and third collected BALF were combined and centrifuged under the same conditions as the first collected BALF. The cells obtained by decanting the supernatant was hit slightly to separate them, to which the cells obtained from the first centrifugation were added. The total cell numbers were determined with 0.5 ml CM-10, wherein the density of cells was adjusted to 3×10⁵ cells/ml. Cytospin centrifuging machine (Cytospin 4 Cytocentrifuge, Thermo Shandon, USA) was used to immobilize the cells for the experiments carried out thereafter. 200 µl of cell suspension per mouse was centrifuged at 500 rpm for four minutes, and a slide was prepared, on which the cell suspension was dried in the air. The cells were stained with Liu's stain solution (Liu A and Liu B, Delta, 232, Japan). The cells were observed with a microscope (Olympus, BX41TF, Japan) equipped with an oil immersion lens under 1000 times of magnification. Two hundreds of leukocytes were counted on each slide, which includes four different cells, eosinophil, neutrophil, lymphocyte and monocyte. The experiment results were presented as percentage of specific sub-population of lymphocytes based on the total BALF cells.
(6) Eotaxin level measurement:
   The Eotaxin level in bronchoalveolar lavage was measured by Sandwich-ELISA with a kit sold under a trademark of R&D. Briefly, ELISA plates were first
   covered with a specific Abs and left over night at 4°C. The plates were treated with 1% PBS-BSA and washed before conducting the experiments. Supernatants were then added to the ELISA plates, which were kept at room temperatures for two hours, followed by adding biotin-conjugated Abs. After two more hours at room temperature, avidin-conjugated HRP was then added and left still for two hours. The substrate tetramethylbenzidine (TMB) was then added for coloring, and the wavelength absorbed was measured at OD450 wavelength. The concentrations were calculated by interpolation based on the standard values.
(7) Biological statistical analysis:
   All data were presented as mean ± SD. All analysis for statistically significant differences was performed with Student's t test as compared with treatment to the As group. *P* values < 0.05 were considered significant.

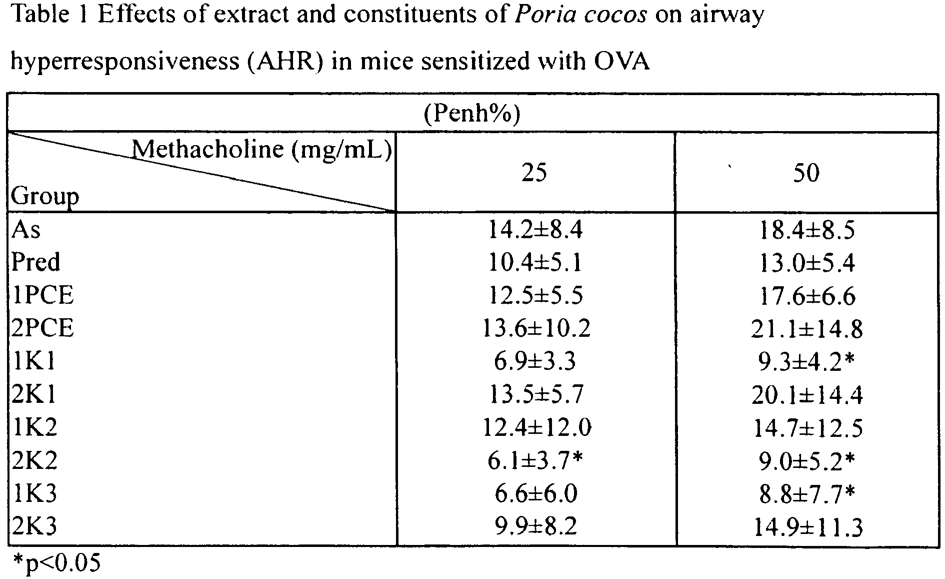

**Table 2 Effects of extract and constituents of Poria cocos on percentage of subpopulation of immune cells in BALF of OVA-sensitized mice**

| | Monocyte | Lymphocyte | Neutrophil | Eosinophil |
|---|---|---|---|---|
| Group | % | | | |
| As | 19.1±7.9 | 64.7±12.2 | 0.6±0.6 | 15.7±8.3 |
| Pred | 13.3±7.8 | 76.4±9.3* | 0.8±0.8 | 9.4±2.5 |
| 1PCE | 16.5±6.5 | 69.4±12.9 | 0.1±0.2* | 15.1±8.0 |
| 2PCE | 14.2±7.7 | 74.7±12.5 | 0.1±0.2* | 11.0±5.8 |
| 1K1 | 15.1±6.6 | 72.1±10.5 | 0±0* | 12.9±6.3 |
| 2K1 | 18.5±10.7 | 67.6±18.8 | 1.0±0.4* | 13.9±9.0 |
| 1K2 | 31.0±6.6 | 57.9±7.2 | 0.4±0.8 | 10.6±3.2 |
| 2K2 | 35.7±7.5 | 50.4±8.2 | 0.6±0.5 | 13.4±3.8 |
| 1K3 | 38.2±8.7 | 49.8±12.8 | 0.8±0.9 | 11.3±7.0 |
| 2K3 | 37.1±5.7 | 52.1±8.1 | 1.1±1.1 | 9.7±3.6 |

| | | | | |
|---|---|---|---|---|
| *p<0.05 | | | | |

**Table 3 Effects of extract and constituents of Poria cocos on levels of eotaxin in BALF of OVA-sensitized mice**

| Group | Eotaxin (pg/mL) |
|---|---|
| As | 34.1±9.3 |
| Pred | 31.1±6.8 |
| 1PCE | 20.9±6.4* |
| 2PCE | 27.1±11.1 |
| 1K1 | 19.6±8.8* |
| 2K1 | 21.5±5.8* |
| 1K2 | 22.1±13.1 |
| 2K2 | 19.3±6.5* |
| 1K3 | 21.1±10.4* |
| 2K3 | 25.1±12.0 |

| | |
|---|---|
| *p<0.05 | |

### Results:

1. It can be seen from Table 1 that, as the concentration of methacholine increases, the value of AHR also increases among groups. The 2K2 group shows the lowest AHR values as compared to the As group, and the 1K1, the 2K2, and the 1K3 groups also have significantly lower values ofAHR in comparison with the As group upon higher stimulation of methacholine (50mg/mL). As the higher values of AHR indicate that the mice suffer from more severity of airway hyperresponsiveness response. The resultant data indicate that the extract and constituents of *Poria cocas* of the present inveniot have preventive/therapeutic effects on asthma. The Pred group shows lower values of AHR but not statistically significant to the As group. Therefore, the extract and constituents of *Poria cocos* of the present invention are more advantageous in preventive/therapeutic effects on asthma in term of the contraction of airway caused by asthma, in comparison with the popular clinical therapeutic steroids for asthma treatment.
2. It can be seen from the results shown in Table 2 that the As group has the highest percentage and the Pred group has the lowest percentage of eosinophil in BALF of OVA-sensitized mice but without statistical significance. However, the percentage of lymphocyte in the Pred group is significantly higher than that of the As group. In spite of that, mice which have been administered with 1PCE, 2PCE, 1K1, and 2K1 show significantly lower percentages of neutrophil in the BALF in comparison with the As group. The results indicate that the constituents of *Poria cocos* can attenuate the infiltration of inflammatory cells sustained in the lung tissue.
3. On the active phase of asthma initiation stages, there are many kinds of inflammatory proteins involved in the local inflammation. In this example, we analyzed the levels of Eotaxin in the BALF, and the results are shown in Table 3. There are many kinds of cells can secrete Eotaxin, such as the epithelial cell on the airway bronchia, the endothelial cells and the fibroblast cells. Secreted Eotaxin can trigger the eosinophils or Th2 cells to airway bronchia (Rankin et al., Eotaxin and eosinophil recruitment: implication for human disease. Mol Med Today 6:20-27.(2000)). It can be seen from data in Table 3 that mice which have been administered with 1PCE, 1K1, 2K1, and 1K3 show significantly lower levels of Eotaxin in BALF in comparison with the As group. As to the mice in the Pred group show no significant difference on the levels of Eotaxin in comparison with the As group. The results support that the constituents of *Poria cocos* disclosed in the present invention can prevent the airway from the infiltration of inflammatory cells by inhibiting the secretion of inflammatory protein Eotaxin. Therefore, the constituents of *Poria cocos* of the present invention are more advantageous in preventive/therapeutic effects on asthma in term of attenuating the infiltration of inflammatory cells (eosinophils or Th2 lymphocytes) sustained in the lung tissue, in comparison with the popular clinical therapeutic steroids (Pred) for asthma treatment.

## Claims

1. A lanostane having the following chemical formula (I) or a pharmaceutically acceptable salt thereof for use in the treatment of allergy in a mammal, 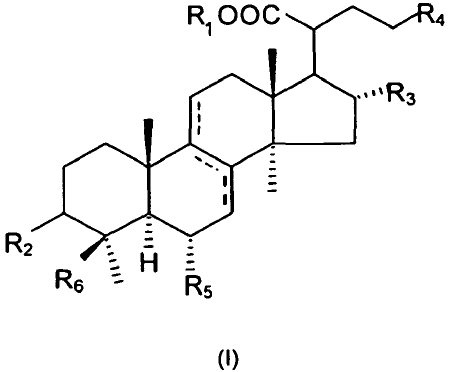 wherein R₁ is either H or CH₃; R₂ is OCOCH₃, =O or OH; R₃ is H or OH; R₄ is -C(=CH₂)-C(CH₃)₂Rₐ, in which Rₐ is H or OH, or -CH=C(CH₃)-R_{b}, in which R_{b} is CH₃ or CH₂OH; R₅ is H or OH; and R₆ is CH₃ or CH₂OH.

2. The lanostane for use according to claim 1, wherein said allergy is allergic rhinitis, allergic conjunctivitis, allergic asthma, atopic dermatitis, gullet allergy, atopic eczema, or rheumatoid arthritis.

3. The lanostane for use according to claim 2, wherein said allergy is allergic asthma.

4. The lanostane for use according to claim 1, wherein the lanostane (I) is 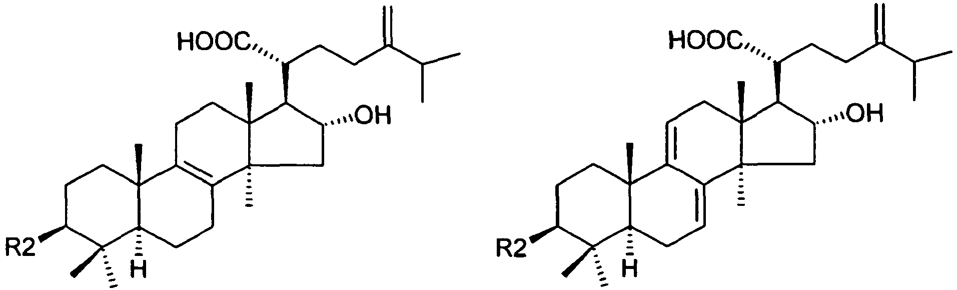 or 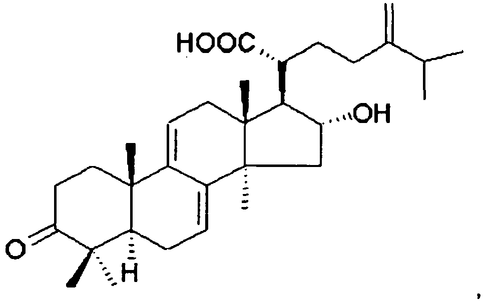 wherein R2 is OCOCH₃ or OH.

5. The lanostane for use according to claim 1, which is contained in a medicament in a range of 0.1-60 wt%.

6. The lanostane for use according to claim 5, which is contained in a medicament for oral administration.

7. The lanostane for use according to claim 1, wherein the mammal is a human.

8. The lanostane for use according to claim 1, which is derived from *Poria* extract, and which comprises 1-60% of the lanostane (I) by weight of the extract, and being substantially devoid of secolanostane.

9. The lanostane for use according to claim 8, wherein said *Poria* extract is prepared by a method comprising the following steps:
a) extracting metabolites, fermentation products or sclerotium of *Poria cocos* (Schw) Wolf by water, methanol, ethanol, or a mixed solvent thereof;
b) concentrating the resulting liquid extract from step a);
c) introducing the resulting concentrated substance from step b) into a silica gel column;
d) eluting the silica gel column with an eluent having a low polarity, and collecting the resulting eluate; and
e) concentrating the eluate to form a concentrated eluate.

10. The lanostane for use according to claim 9, wherein the concentrated eluate from step e) has a chromatographic value, Rf, not less than 0.1 in accordance with a thin layer chromatography, which is developed by a mixed solvent of dichloromethane : methanol = 96:4 and is detected by an ultraviolet lamp and iodine vapor.

11. The lanostane for use according to claim 9, wherein the extraction in step a) is carried out by using 95% ethanol.

12. The lanostane for use according to claim 9, wherein the extraction in step a) comprises extracting metabolites, fermentation products or sclerotium of *Poria cocos* (Schw) Wolf by boiling water; adding a base to the resulting extraction aqueous solution until a pH value thereof is 9-11; recovering the basic aqueous solution; adding an acid to the basic aqueous solution until a pH value thereof is 4-6 to form a precipitate; recovering the precipitate; extracting the precipitate with ethanol; and recovering a liquid extract.

13. The lanostane for use according to claim 11 or 12, wherein the concentrated substance resulted from step b) is further extracted with a two-phase solvent containing methanol and n-hexane in a volumetric ratio of 1:1, a methanol layer is separated from the two-phase solvent extraction mixture, and the methanol layer is concentrated to form a concentrate, which is used as a feed to the silica gel column in step c).

14. The lanostane for use according to claim 9, wherein the low polarity eluent in step d) is a mixed solvent containing dichloromethane and methanol in a volumetric ratio of 96.5:3.5.

15. The lanostane for use according to claim 8, wherein said *Poria* extract comprises 5-35% of the lanostane (I).

16. The lanostane for use according to claim 8, wherein the lanostane (I) is 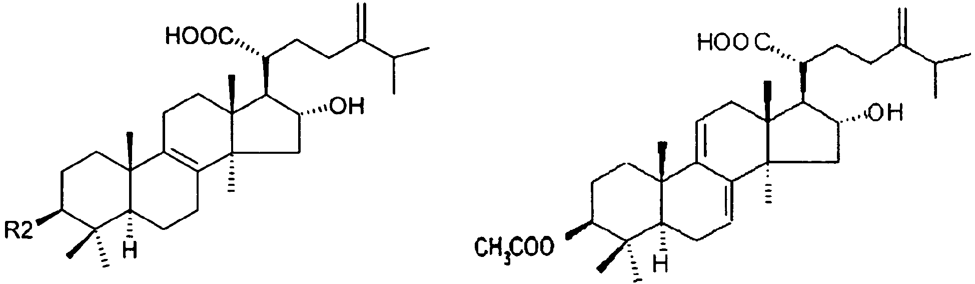 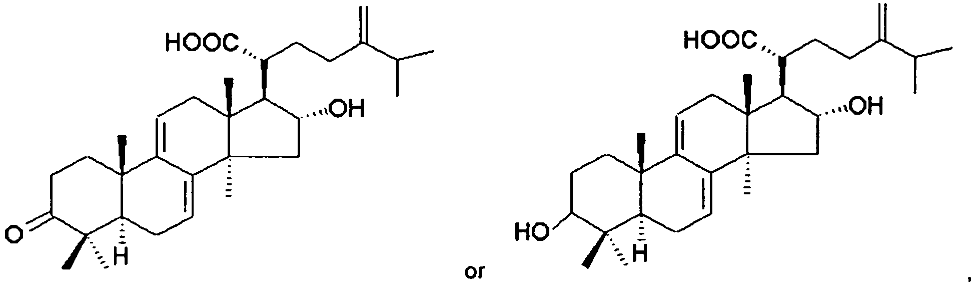 wherein R2 is OCOCH₃ or OH.

## Patentansprüche

1. Lanostan mit der folgenden chemischen Formel (I) oder ein pharmazeutisch verträgliches Salz davon zur Verwendung bei der Behandlung einer Allergie in einem Säuger, 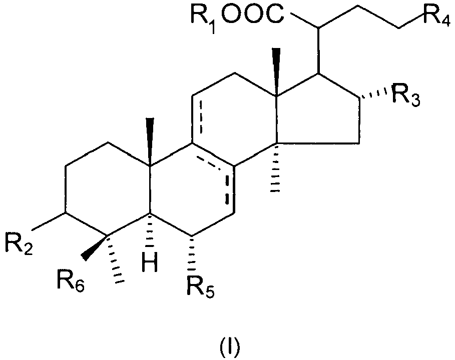 wobei R₁ entweder H oder CH₃ ist; R₂ OCOCH₃, =O oder OH ist; R₃ H oder OH ist; R₄ -C(=CH₂)-C(CH₃)₂Rₐ, in welchem Rₐ H oder OH ist, oder -CH=C(CH₃)-R_{b} ist, in welchem R_{b} CH₃ oder CH₂OH ist; R₅ H oder OH ist; und R₆ CH₃ oder CH₂OH ist.

2. Lanostan zur Verwendung gemäß Anspruch 1, wobei es sich bei der Allergie um allergische Rhinitis, allergische Konjunktivitis, allergisches Asthma, atopische Dermatitis, Speiseröhrenallergie, atopisches Ekzem oder rheumatoide Arthritis handelt.

3. Lanostan zur Verwendung gemäß Anspruch 2, wobei es sich bei der Allergie um allergisches Asthma handelt.

4. Lanostan zur Verwendung gemäß Anspruch 1, wobei das Lanostan (I) 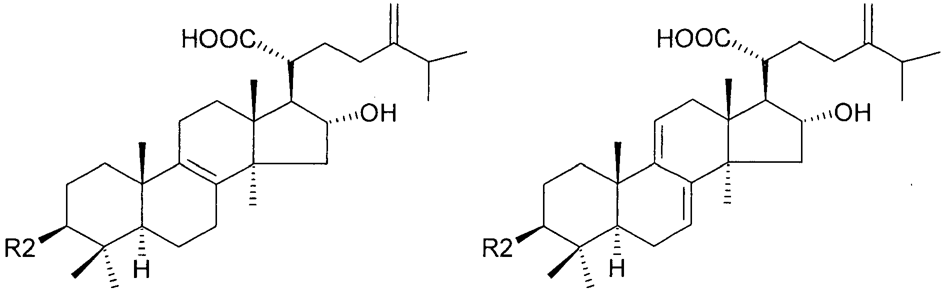 oder 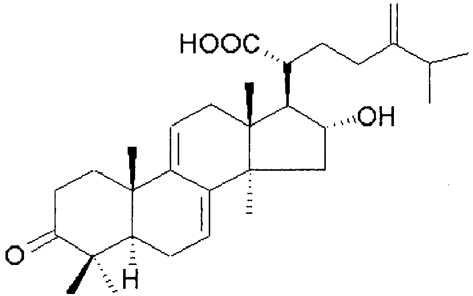 ist, wobei R2 OCOCH₃ oder OH ist.

5. Lanostan zur Verwendung gemäß Anspruch 1, welches in einem Medikament in einem Bereich von 0,1 - 60 Gew.-% enthalten ist.

6. Lanostan zur Verwendung gemäß Anspruch 5, welches in einem Medikament zur oralen Verabreichung enthalten ist.

7. Lanostan zur Verwendung gemäß Anspruch 1, wobei der Säuger ein Mensch ist.

8. Lanostan zur Verwendung gemäß Anspruch 1, welches aus einem Poria-Extrakt gewonnen ist, und welches 1 - 60 % des Lanostans (I), bezogen auf das Gewicht des Extrakts, umfasst und welches im Wesentlichen frei von Secolanostan ist.

9. Lanostan zur Verwendung gemäß Anspruch 8, wobei der Poria-Extrakt durch ein Verfahren hergestellt wird, das die folgenden Schritte umfasst:
a) Extrahieren von Metaboliten, Fermentationsprodukten oder einem Sklerotium von *Poria cocos* (Schw) Wolf durch Wasser, Methanol, Ethanol oder ein gemischtes Lösungsmittel davon;
b) Konzentrieren des resultierenden flüssigen Extrakts von Schritt a);
c) Einbringen der resultierenden konzentrierten Substanz von Schritt b) in eine Silicagelsäule;
d) Eluieren der Silicagelsäule mit einem Elutionsmittel mit einer niedrigen Polarität, und Sammeln des resultierenden Eluats; und
e) Konzentrieren des Eluats, um ein konzentriertes Eluat zu bilden.

10. Lanostan zur Verwendung gemäß Anspruch 9, wobei das konzentrierte Eluat von Schritt e) einen chromatografischen Wert Rf von nicht weniger als 0,1 aufweist, gemäß einer Dünnschichtchromatografie, welche durch ein gemischtes Lösungsmittel aus Dichlormethan : Methanol = 96 : 4 entwickelt wird und durch eine Ultraviolettlampe und loddampf nachgewiesen wird.

11. Lanostan zur Verwendung gemäß Anspruch 9, wobei die Extraktion in Schritt a) unter Verwendung von 95 % Ethanol durchgeführt wird.

12. Lanostan zur Verwendung gemäß Anspruch 9, wobei die Extraktion in Schritt a) das Extrahieren von Metaboliten, Fermentationsprodukten oder einem Sklerotium von *Poria cocos* (Schw) Wolf durch siedendes Wasser; das Zugeben einer Base zu der resultierenden wässrigen Extraktionslösung, bis ein pH-Wert davon 9 - 11 beträgt; das Gewinnen der basischen wässrigen Lösung; das Zugeben einer Säure zu der basischen wässrigen Lösung, bis ein pH-Wert davon 4 - 6 beträgt, um einen Niederschlag zu bilden; das Gewinnen des Niederschlags; das Extrahieren des Niederschlags mit Ethanol; und das Gewinnen eines flüssigen Extrakts umfasst.

13. Lanostan zur Verwendung gemäß Anspruch 11 oder 12, wobei die sich aus Schritt b) ergebende konzentrierte Substanz mit einem Zweiphasenlösungsmittel, das Methanol und n-Hexan in einem volumetrischen Verhältnis von 1 : 1 enthält, weiter extrahiert wird, eine Methanolschicht von der Zweiphasenlösungsmittel-Extraktionsmischung abgetrennt wird, und die Methanolschicht konzentriert wird, um ein Konzentrat zu bilden, welches als ein Aufgabegut für die Silicagelsäule in Schritt c) verwendet wird.

14. Lanostan zur Verwendung gemäß Anspruch 9, wobei das Elutionsmittel mit niedriger Polarität in Schritt d) ein gemischtes Lösungsmittel ist, das Dichlormethan und Methanol in einem volumetrischen Verhältnis von 96,5 : 3,5 enthält.

15. Lanostan zur Verwendung gemäß Anspruch 8, wobei der Poria-Extrakt 5 - 35 % des Lanostans (I) umfasst.

16. Lanostan zur Verwendung gemäß Anspruch 8, wobei das Lanostan (I) 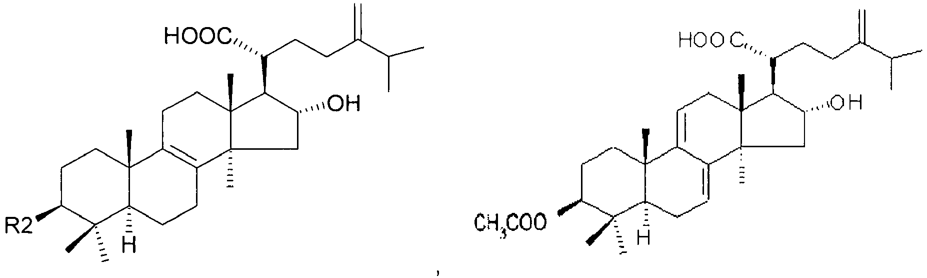 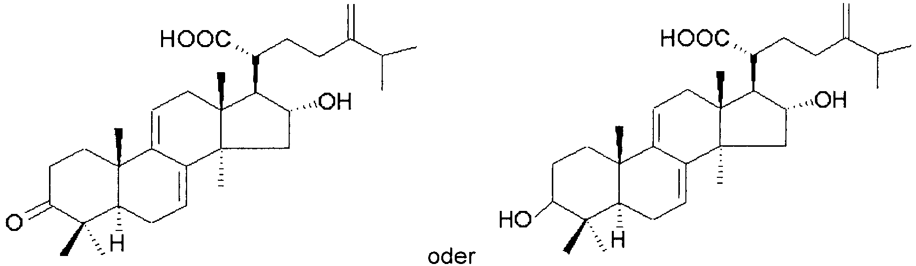 ist,
wobei R2 OCOCH₃ oder OH ist.

## Revendications

1. Lanostane répondant à la formule chimique (I) suivante ou sel pharmaceutiquement acceptable de celui-ci destiné à être utilisé dans le traitement d'une allergie chez un mammifère, 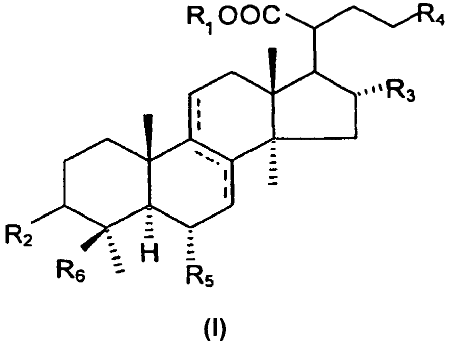 dans laquelle R₁ est soit H, soit CH₃ ; R₂ est OCOCH₃, =O ou OH ; R₃ est H ou OH ; R₄ est -C(=CH₂)-C(CH₃)₂Rₐ, où Rₐ est H ou OH, ou -CH=C(CH₃)-R_{b}, où R_{b} est CH₃ ou CH₂OH ; R₅ est H ou OH ; et R₆ est CH₃ ou CH₂OH.

2. Lanostane destiné à être utilisé selon la revendication 1, ladite allergie étant une rhinite allergique, une conjonctivite allergique, un asthme allergique, une dermatite atopique, une allergie oesophagienne, un eczéma atopique ou une arthrite rhumatoïde.

3. Lanostane destiné à être utilisé selon la revendication 2, ladite allergie étant un asthme allergique.

4. Lanostane destiné à être utilisé selon la revendication 1, le lanostane (I) étant 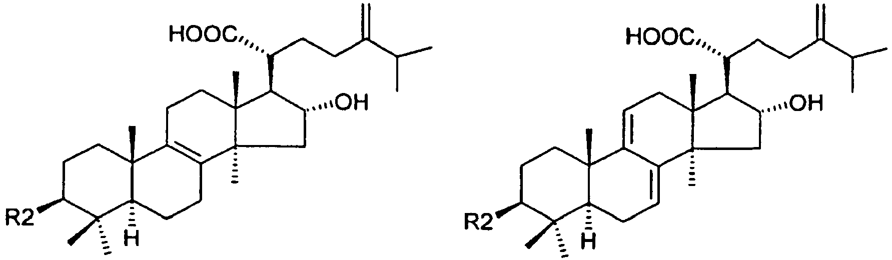 ou 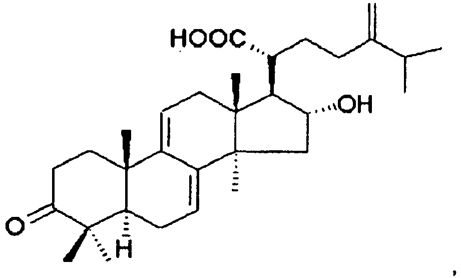 dans lesquelles R2 est OCOCH₃ ou OH.

5. Lanostane destiné à être utilisé selon la revendication 1, lequel est présent dans un médicament dans une fourchette de 0,1 à 60 % en poids.

6. Lanostane destiné à être utilisé selon la revendication 5, lequel est présent dans un médicament pour administration par voie orale.

7. Lanostane destiné à être utilisé selon la revendication 1, le mammifère étant un être humain.

8. Lanostane destiné à être utilisé selon la revendication 1, lequel est dérivé d'un extrait de *Poria,* et le lanostane (I) constituant de 1 à 60 % en poids de l'extrait et étant sensiblement dépourvu de secolanostane.

9. Lanostane destiné à être utilisé selon la revendication 8, ledit extrait de *Poria* étant préparé par un procédé comprenant les étapes suivantes :
a) extraction de métabolites, produits de fermentation ou sclérote de *Poria cocos* (Schw) Wolf au moyen d'eau, de méthanol, d'éthanol ou d'un solvant mixte les comprenant ;
b) concentration de l'extrait liquide résultant issu de l'étape a) ;
c) introduction de la substance concentrée résultante issue de l'étape b) dans une colonne de gel de silice ;
d) élution de la colonne de gel de silice au moyen d'un éluant de faible polarité, et collecte de l'éluat résultant ;
e) concentration de l'éluat pour former un éluat concentré.

10. Lanostane destiné à être utilisé selon la revendication 9, l'éluat concentré issu de l'étape e) ayant un rapport frontal, Rf, non inférieur à 0,1 d'après une chromatographie sur couche mince, laquelle est menée à bien au moyen d'un solvant mixte contenant dichlorométhane et méthanol dans un rapport de 96:4 et révélée au moyen d'une lampe ultraviolette et de vapeur d'iode.

11. Lanostane destiné à être utilisé selon la revendication 9, l'extraction menée à l'étape a) étant réalisée en utilisant 95 % d'éthanol.

12. Lanostane destiné à être utilisé selon la revendication 9, l'extraction menée à l'étape a) comprenant l'extraction de métabolites, produits de fermentation ou sclérote de *Poria cocos* (Schw) Wolf au moyen d'eau bouillante ; l'ajout d'une base à la solution aqueuse d'extraction résultante jusqu'à ce qu'une valeur de pH de celle-ci soit de 9 à 11 ; la récupération de la solution aqueuse basique ; l'ajout d'un acide à la solution aqueuse basique jusqu'à ce qu'une valeur de pH de celle-ci soit de 4 à 6 pour former un précipité ; la récupération du précipité ; l'extraction du précipité à l'éthanol ; et la récupération d'un extrait liquide.

13. Lanostane destiné à être utilisé selon la revendication 11 ou 12, la substance concentrée issue de l'étape b) étant soumise à une extraction supplémentaire au moyen d'un solvant bi-phasique contenant du méthanol et du n-hexane dans un rapport 1:1 en volume, une couche de méthanol étant séparée du mélange d'extraction au solvant bi-phasique, et la couche de méthanol étant concentrée afin de former un concentré, lequel est employé en tant qu'alimentation de la colonne de gel de silice à l'étape c).

14. Lanostane destiné à être utilisé selon la revendication 9, l'éluant de faible polarité de l'étape d) étant un solvant mixte contenant du dichlorométhane et du méthanol dans un rapport de 96,5:3,5 en volume.

15. Lanostane destiné à être utilisé selon la revendication 8, ledit extrait de *Poria* comprenant de 5 à 35 % du lanostane (I).

16. Lanostane destiné à être utilisé selon la revendication 8, le lanostane (I) étant 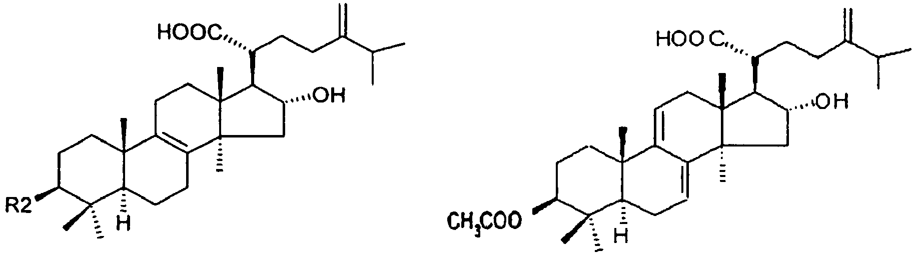 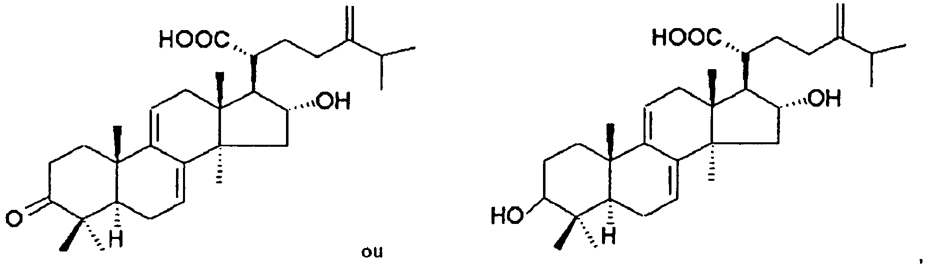 dans lesquelles R2 est OCOCH₃ ou OH.
